**Europäisches Patentamt**

**European Patent Office**

**Office européen des brevets**

(11) Veröffentlichungsnummer: **0 087 597**
**B1**

(12) **EUROPÄISCHE PATENTSCHRIFT**

(45) Veröffentlichungstag der Patentschrift:
16.01.85

(51) Int. Cl.⁴: **C 07 C 13/48**, C 07 C 5/11,
B 01 J 23/74

(21) Anmeldenummer: 83100866.9

(22) Anmeldetag: 31.01.83

(54) **Verfahren zur Herstellung von Tetralin durch selektive Hydrierung von Naphthalin.**

(30) Priorität: 12.02.82 DE 3205102

(43) Veröffentlichungstag der Anmeldung:
07.09.83 Patentblatt 83/36

(45) Bekanntmachung des Hinweises auf die Patenterteilung:
16.01.85 Patentblatt 85/3

(84) Benannte Vertragsstaaten:
BE DE FR IT

(56) Entgegenhaltungen:
DE - A - 2 229 844
DE - B - 1 518 558
US - A - 3 541 169
US - A - 4 003 956
US - A - 4 224 458

(73) Patentinhaber: EC ERDÖLCHEMIE GMBH,
Postfach 75 20 02, D-5000 Köln 71 (DE)

(72) Erfinder: Schleppinghoff, Bernhard, Dr.,
Adolf-Kolping-Strasse 5, D-4047 Dormagen 1 (DE)
Erfinder: Sinhuber, Albrecht, Dr., Scheibenstrasse 26,
D-4000 Duesseldorf 1 (DE)
Erfinder: Mentzen, Heinz, Herriger Weg 45,
D-5021 Sinnersdorf (DE)

(74) Vertreter: Mann, Volker, Dr. et al, c/o Bayer
Aktiengesellschaft Zentralbereich Patente Marken und
Lizenzen, D-5090 Leverkusen-Bayerwerk (DE)

## Beschreibung

Die vorliegende Erfindung betrifft ein Verfahren zur selektiven Hydrierung von Naphthalin zum Tetralin in einem Lösungsmittel an einem Nickelkatalysator unter milden Bedingungen.

Tetralin ist ein gutes Lösungsmittel für Harze und Lacke sowie ein wertvoller Ausgangsstoff für verschiedene chemische Produkte. In letzter Zeit findet Tetralin zunehmendes Interesse bei der Kohlehydrierung als Wasserstoffüberträger.

Eine Übersicht über ältere Herstellungsverfahren von Tetralin ist in „Ullmanns Enzyklopädie der technischen Chemie", 3. Aufl., Bd. 12, S. 589 (1960) enthalten. Als Ausgangsstoff wird Naphthalin von verschieden hoher Reinheit ohne Zusatz eines Lösungsmittels eingesetzt.

Nach einem neueren Verfahren der US Nr. 3541169 wird Naphthalin in 4 bis 7 hintereinander geschalteten Festbettreaktoren bei 210 bis 290°C stufenweise zum Tetralin hydriert. Als Katalysator dient ein Nickelkontakt mit 0,5 bis 25% Nickeloxid (entspricht etwa 0,4 bis 19,6% Ni) auf einem festen Träger. Ein höherer Nickelgehalt als der genannte bewirkt nach dieser Patentschrift eine Herabsetzung der Selektivität.

Die Hydrierung des Naphthalins zum Tetralin mit Hilfe komplexer anionischer Hydride von Metallen der VIII. Gruppe des Periodensystems bei Temperaturen bis 150°C wird in US Nr. 4224458 beschrieben.

In der DE-OS Nr. 1518558 wird ein Verfahren zur Hydrierung von Naphthalin zum Tetralin beschrieben, das bei hohen Drücken von 40 bis 300 bar und Temperaturen von bis zu 300°C an einem komplexen Katalysatorsystem, das Nickel, Kobalt, Molybdän und Wolfram enthält, arbeitet.

Der Nachteil der bisherigen Verfahren zur Herstellung von Tetralin durch selektive Hydrierung des Naphthalins liegt in der Verwendung teurer und komplizierter Katalysatorsysteme, in der Notwendigkeit hoher Wasserstoffdrücke und im Einsatz mehrerer hintereinandergeschalteter Hydrierreaktoren, wobei zur Erreichung hoher Selektivitäten nur Nickelkontakte bis unterhalb 20 Gew.-% Ni auf einem festen Träger eingesetzt werden können.

Es wurde nun ein Verfahren zur selektiven katalytischen Hydrierung von Naphthalin zum Tetralin bei erhöhter Temperatur mit Wasserstoff an einem Nickel-Träger-Katalysator gefunden, das dadurch gekennzeichnet ist, dass der Katalysator 20 bis 60 Gew.-% Nickel, bezogen auf das Gesamtgewicht des Katalysators, hat und die Reaktion in einem Lösungsmittel bei 150 bis 250°C und einem Druck von 1 bis 20 bar durchgeführt wird.

Als Einsatzprodukt für das erfindungsgemässe Verfahren wird reines Naphthalin mit einem Gehalt von nur wenigen ppm Schwefel, beispielsweise bis zu 30 ppm, verwendet. Ein solches schwefelarmes Naphthalin lässt sich beispielsweise aus dem Crackmittelöl eines Steamcrackers gewinnen. Selbstverständlich können auch Naphthaline anderer Herkunft verwendet werden, wenn sie vorher einer geeigneten Entschwefelung unterzogen werden.

Als Lösungsmittel im erfindungsgemässen Verfahren eignen sich cyclische paraffinische Kohlenwasserstoffe, wie Cyclohexan, Methylcyclohexan, Dimethylcyclohexan, Ethylcyclohexan, Cyclopentan, Methylcyclopentan und andere. Sie werden in einer solchen Menge eingesetzt, dass die Naphthalin-Lösung eine Konzentration von 10 bis 60 Gew.-% Naphthalin, bezogen auf die gesamte Lösung, hat. Das Lösungsmittel begünstigt den Wärmeübergang und garantiert eine gleichmässige Reaktionsführung.

Erfindungsgemäss wird ein Nickel-Träger-Kontakt mit 20 bis 60 Gew.-% Nickel, bevorzugt 25 bis 45 Gew.-% Nickel, bezogen auf das Gesamtgewicht des Katalysators, eingesetzt. Als Träger kommen beispielsweise $SiO_2$ oder $Al_2O_3$ in Frage. In bevorzugter Weise haben solche Träger eine innere Oberfläche von 50 bis 250 m²/g, vorzugsweise 100-180 m²/g, und ein Porenvolumen von 0,2 bis 0,55 ml/g, vorzugsweise 0,3-0,4 ml/g. Besonders vorteilhaft als Träger erweist sich ein $SiO_2$ mit den genannten Eigenschaften, beispielsweise Kieselgur.

Ein solcher Nickel-Träger-Kontakt kann beispielsweise nach der Methode der Tränkung des Trägers mit einer Nickelsalzlösung und anschliessender thermischer Behandlung hergestellt werden.

Der fertige Katalysator wird vor der Hydrierungsreaktion mit Wasserstoff bei höheren Temperaturen von etwa 150 bis 300°C aktiviert.

Das erfindungsgemässe Verfahren kann beispielsweise kontinuierlich in einem Festbettreaktor in der Flüssigphase oder in der Rieselphase durchgeführt werden. Zur Hydrierung kann reiner oder technischer Wasserstoff, der beispielsweise bis zu 20 Vol.-% Methan enthält, eingesetzt werden.

Die Reaktion wird bei einem Druck von 1 bis 20 bar, bevorzugt 2 bis 10 bar, und einer Temperatur von 150 bis 250, bevorzugt 180 bis 220°C, durchgeführt. Die Naphthalinlösung in einem der genannten Lösungsmittel wird mit einer WHSV (= *weight, hourly space velocity*) von 0,3 bis 10 pro Stunde an dem genannten Trägerkatalysator vorbeigeführt.

Das den Reaktor verlassende Reaktionsprodukt wird in 2 Destillationsstufen aufgearbeitet. Das im Kopf der ersten Kolonne anfallende reine Lösungsmittel wird in das erfindungsgemässe Verfahren zurückgeführt. Aus dem Sumpfprodukt der ersten Kolonne, das nur noch Tetralin und geringe Mengen an Dekalin und Naphthalin enthält, wird in der zweiten Kolonne ein Tetralin mit einer Reinheit von mehr als 99% gewonnen. Im erfindungsgemässen Verfahren wird das eingesetzte Naphthalin in nur einem Durchgang zu mehr als 92% umgesetzt, wobei eine Selektivität von mehr als 95% erreicht wird.

*Beispiel 1:*

In einem röhrenförmigen, beheizbaren Reaktor mit einem Volumen von 300 ml befindet sich ein fester Nickelkontakt mit 35 Gew.-% Nickel auf einem SiO₂-(Kieselgur)-Träger. Die Oberfläche des Kontaktes beträgt 120 m²/g und das Porenvolumen 0,4 ml/g. Der Kontakt wird 2 h mit Wasserstoff bei 200°C und Atmosphärendruck aktiviert.

Über den aktivierten Kontakt wird eine Lösung von 20 Gew.-% Naphthalin in Cyclohexan bei einer Temperatur von 208°C, einem Druck von 4,5 bar und einer WHSV von 1,3, bezogen auf die verdünnte Lösung, gefahren. Die Lösung enthält 5 ppm Schwefel. Das Molverhältnis Wasserstoff zu Naphthalin beträgt 3:1. Es wird ein Umsatz von 95% und eine Selektivität von 97,1% erhalten.

*Beispiel 2:*

Über den Kontakt wie in Beispiel 1 wird eine 22%ige Lösung von Naphthalin in Cyclohexan bei einer Temperatur von 210°C und einem Druck von 6 bar mit einer WHSV von 1,7 gefahren. Die Lösung enthält 4 ppm Schwefel.

Das Molverhältnis Wasserstoff zu Naphthalin beträgt 4:1. Es wird ein Umsatz von 92,2% und eine Selektivität von 97,5% erhalten.

## Patentansprüche

1. Verfahren zur selektiven katalytischen Hydrierung von Naphthalin zum Tetralin bei erhöhter Temperatur mit Wasserstoff an einem Nickel-Träger-Katalysator, dadurch gekennzeichnet, dass der Katalysator 20 bis 60 Gew.-% Nickel, bezogen auf das Gesamtgewicht des Katalysators, hat und die Reaktion in einem Lösungsmittel bei 150 bis 250°C und einem Druck von 1 bis 20 bar durchgeführt wird.

2. Verfahren nach Anspruch 1, dadurch gekennzeichnet, dass der Katalysator 25 bis 45 Gew.-% Nickel enthält.

3. Verfahren nach den Ansprüchen 1 und 2, dadurch gekennzeichnet, dass als Träger SiO₂ oder Al₂O₃ eingesetzt werden.

4. Verfahren nach den Ansprüchen 1 bis 3, dadurch gekennzeichnet, dass der Träger eine innere Oberfläche von 50 bis 250 m²/g und ein Porenvolumen von 0,2 bis 0,55 ml/g hat.

5. Verfahren nach den Ansprüchen 1 bis 4, dadurch gekennzeichnet, dass Naphthalin in einem cyclischen paraffinischen Lösungsmittel eingesetzt wird.

6. Verfahren nach den Ansprüchen 1 bis 5, dadurch gekennzeichnet, dass die Reaktionstemperatur 180 bis 220°C beträgt.

7. Verfahren nach den Ansprüchen 1 bis 6, dadurch gekennzeichnet, dass der Druck 2 bis 10 bar beträgt.

8. Verfahren nach den Ansprüchen 1 bis 7, dadurch gekennzeichnet, das die WHSV (= *weight, hourly space velocity*) 0,3 bis 10 pro Stunde, bezogen auf die Lösung des Naphthalins im Lösungsmittel, beträgt.

## Claims

1. Process for the selective catalytic hydrogenation of naphthalene to give tetraline at an elevated temperature with hydrogen on a nickel supported catalyst, characterised in that the catalyst has 20 to 60% by weight of nickel, based on the total weight of the catalyst, and the reaction is carried out in a solvent at 150 to 250°C and under a pressure of 1 to 20 bar.

2. Process according to Claim 1, characterised in that the catalyst contains 25 to 45% by weight of nickel.

3. Process according to Claims 1 and 2, characterised in that SiO₂ or Al₂O₃ are used as the support.

4. Process according to Claims 1 to 3, characterised in that the support has an inner surface of 50 to 250 m²/g and a pore volume of 0.2 to 0.55 ml/g.

5. Process according to Claims 1 to 4, characterised in that naphthalene in a cyclic paraffinic solvent is used.

6. Process according to Claims 1 to 5, characterised in that the reaction temperature is 180 to 220°C.

7. Process according to Claims 1 to 6, characterised in that the pressure is 2 to 10 bar.

8. Process according to Claims 1 to 7, characterised in that the WHSV (= *weight, hourly space velocity*) is 0.3 to 10 per hour, based on the solution of the naphthalene in the solvent.

## Revendications

1. Procédé d'hydrogénation catalytique sélective de naphtalène en tétraline à température élevée avec de l'hydrogène sur un catalyseur à base de nickel fixé sur un support, caractérisé en ce que le catalyseur contient 20 à 60% en poids de nickel par rapport au poids total du catalyseur et en ce que la réaction est conduite dans un solvant à 150-250°C et sous une pression de 1 à 20 bar.

2. Procédé suivant la revendication 1, caractérisé en ce que le catalyseur contient 25 à 45% en poids de nickel.

3. Procédé suivant les revendications 1 et 2, caractérisé en ce qu'on utilise comme support SiO₂ ou Al₂O₃.

4. Procédé suivant les revendications 1 à 3, caractérisé en ce que le support présente une surface interne de 50 à 250 m²/g et a un volume des pores de 0,2 à 0,55 ml/g.

5. Procédé suivant les revendications 1 à 4, caractérisé en ce que du naphtalène est utilisé dans un solvant paraffinique cyclique.

6. Procédé suivant les revendications 1 à 5, caractérisé en ce que la température de réaction s'élève à 180-220°C.

7. Procédé suivant les revendications 1 à 6, caractérisé en ce que la pression s'élève à 2-10 bar.

8. Procédé suivant les revendications 1 à 7, caractérisé en ce que la WHSV (= *weight, hourly space velocity*) est de 0,3 à 10 par heure, par rapport à la solution du naphtalène dans le solvant.